# EUROPEAN PATENT APPLICATION

(11) **EP 2 703 001 A1**
(43) Date of publication of application: **05.03.2014**
(21) Application number: 12181821.5
(22) Date of filing: 26.08.2012
(51) Int. Cl.: A61K 35/74, A61K 39/02, A61K 39/05, A61K 41/00

(54) **Tumor vaccination**

(71) Applicant: XAX Kft., 2071 Páty (HU)
(72) Inventor: Andocs, Gabor, 2030 Erd (HU); Szász, Andras, 2071 Páty (HU); Iluri, Nora, Los Gatos, CA California 95032 (US); Szász, Olivér, 2071 Páty (HU)
(74) Representative: Arth, Hans-Lothar

(57) **Abstract**

The present invention relates to a vaccine composed of at least one immune stimulant and radiofrequency waves using capacitive coupling for treatment of primary cancer and its metastases even in disseminated cell-states, which can not be detected by presently available imaging methods or for prevention of relapse of the cancer disease.

## Description

The present invention relates to a vaccine composed of at least one immune stimulant and radiofrequency waves using capacitive coupling for treatment of primary cancer and its metastases even in disseminated cell-states, which can not be detected by presently available imaging methods or for prevention of relapse of the cancer disease.

The present invention uses the patient's own, unique tumor specific antigens. However, these antigens are not recognizable - they are hidden - for the immune cells and consequent reactions. This prevents the malignancy against the immune attack, and the body recognize the tumor as its own tissue-reparation. The immune system is silenced against tumor cells. The present invention provides a vaccine which is able to make free the tumor antigens for recognizing these by the antigen presenting cells (APCs) to start specific immune reaction against the malignancy. It is a special effect provided by the inventive vaccine, namely exposing the hidden antigens, promoting the recognition by APCs (especially dendritic cells) and, preparing them and the full system to build up a specific immune reaction to eradicate the tumor. From this point the vaccine of the present invention generates a natural process.

In short, the immunogenic cell-death caused by the vaccine of the present invention causes activation of the adaptive immune system to fight against the cancer cells. The immune stimulant as a component of the inventive vaccine supports this fight so that the effect is completely systemic having long-term memory.

### Background of the invention

### Radio Frequency Ablation (RFA)

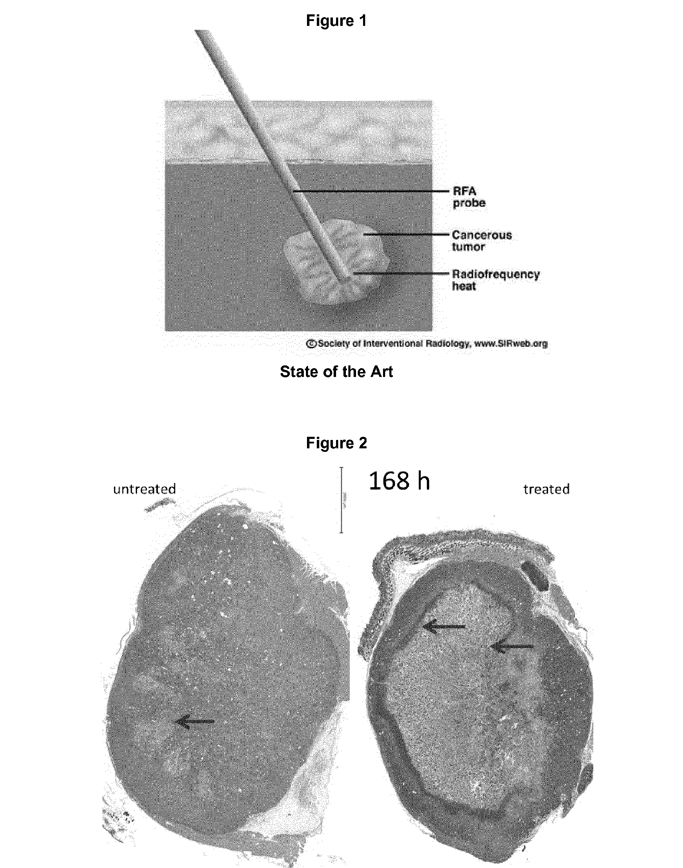

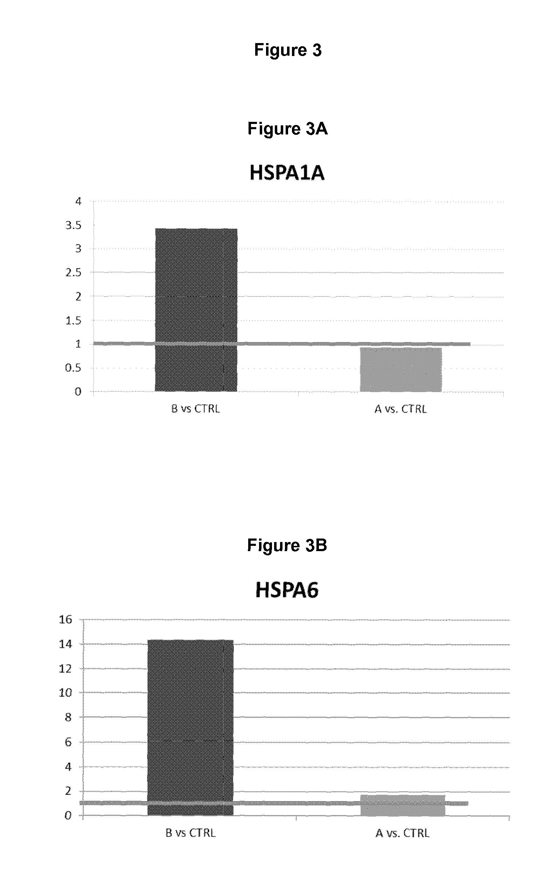

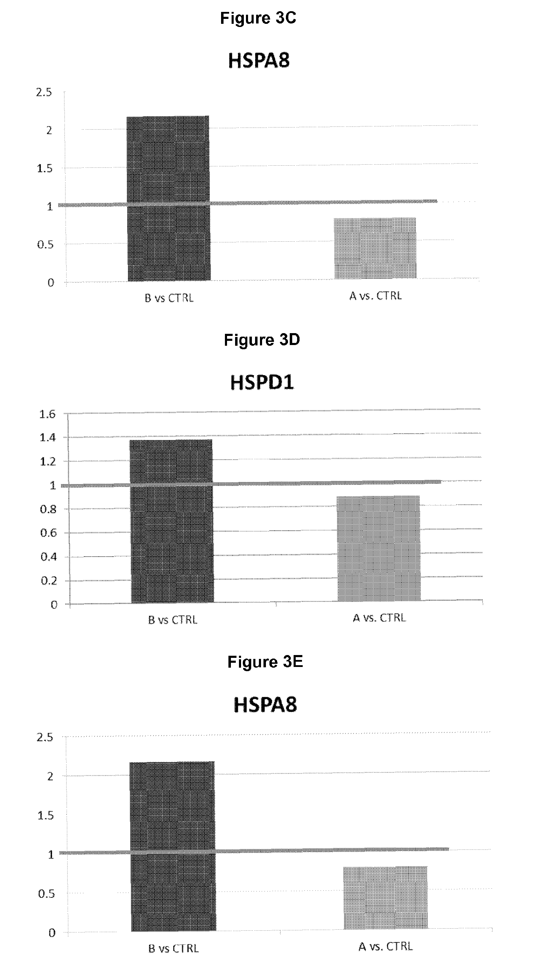

A state of the art method which uses radiofrequency current for the treatment of cancer is the RFA method (radiofrequency ablation) which is quite different from the present invention. The RFA method is an invasive method which uses RF for burning-out the lesion. The RF ablation method applies "antennas" in form of needles (see Figure 1), which are inserted intratumoral (interventional-radiologist guides it by ultrasound or other imaging methods) into the solid tumor, and the applied local current becomes so large, that the tumor is burned thereby causing vehement necrosis. Such conditions are strictly avoided by the present invention. The RF ablation method is a medical procedure, where part of the electrical conduction system of the heart, tumor or other dysfunctional tissue is ablated using the heat generated from the high frequency alternating current to treat a medical disorder. An advantage of RF current (over previously used low frequency AC or pulses of DC) is that it does not directly stimulate nerves or heart muscle and can therefore often be used without the need for general anesthetic. RFA procedures are performed under image guidance (such as X-ray screening, CT scan or ultrasound) by an interventional pain specialist (such as an anesthesiologist), interventional radiologist or a cardiac electrophysiologist, a subspecialty of cardiologists.

### Immune system and cancer

The immune system is a complex structure and its processes protect the organism against irregularities and diseases. It has two basic subsystems, the innate immune system and the adaptive one. The innate immune system is found in almost all the living objects. This has no immune-memory and the action (response) is non-specific and immediate. The main cellular structures of innate immune system are the macrophages (able to phagocytize), mast cells (releasing inflammatory promoters); granulocytes (a group of three cell-types responding to inflammation), dendritic cells (adaptive immune-cells, presenting antigens), natural killer cells (destroy cells infected with pathogens). The adaptive system found in gnathostomatas (vertebrates with jaw) has an immunological memory. It usually has a lag-time for response. The main groups of cells belonging to the adaptive system are B-cells (produce antibodies to neutralize invaders), T-cells (one group thereof destroys the infected cells, another group coordinates the immune-response).

One of the roles of immune system is to identify and destroy the tumors. The recognition is possible with the tumor specific antigens or the tumor associated antigens on tumor cells, which are not expressed on healthy cells. Specific immune response to tumor cells uses T-cells. Some tumors however go to be cancerous, evading the immune system. Tumor cells often express a reduced number of recognizable structures or even hide the recognizable structures which could be recognized by APCs. Some tumors inhibit the immune-response, like for example secreting TGF-β. Additionally immunological tolerance can be developed and no further immune-reaction acts against the cancer. Tumor can make paradoxes also, like macrophages promoting tumor growth in some cases.

A basic theoretical formulation propose a cancer immuno-surveillance, blocking the carcinogenesis and keeping in force the cellular homeostasis. The process when the individual is protected against cancer growth by immune system is the immuno-editing.

Inflammation could be one of the major promoters of tumor-development in elderly subjects.

Present cancer therapies are dominantly focused on the so called "gold standards", such as chemotherapies (pharmaceutical products), radiotherapy (ionizing beams), surgery and their combinations. New methods are emerging, among those have improving position the immune-therapy.

In chemo-thermo-therapies the role of chaperone proteins is important. Chaperones (stress- or heat-shock-proteins) are highly conserved proteins, which are present in almost every living cells. These are like cellular immunecomponents, presented intracellular during their whole lifetime, regardless their stage in the evolution. Any kind of change the dynamic equilibrium of the cell life (environmental stresses, various pathogen processes, diseases, etc.) activates their synthesis. Excretion of the chaperones is the 'stress-answer' of the cells to accommodate themselves to the new challenges. As a consequence of the stressful 'life' of malignant cells, the molecular chaperones are present in all the cancerous cells to adapt the actual stress to help tumor-cell survival. Moreover the shock-proteins are induced by every oncological treatment-method, which are devoted to eliminate the malignancy: After conventional hyperthermia, after chemotherapy, after radiotherapy or even after photodynamic-therapy the intensive HSP synthesis was detected. On the way of the stress adaptation the induction or overexpression of the stress proteins generally provide effective protection of the cell against apoptosis, but their extracellular expression acts oppositely. It makes signal to the immune system on the defect of the actual cell. Furthermore, induction of various HSPs (HSP27, HSP70, and HSP90) was observed in numerous metastases and the HSP90 homologue, GRP94 may act as a mediator of metastasis generation. HSP generally degrades the effect of the hyperthermia therapy, because it may increase the tumor cell survival, and its massive induction may generate the tumor thermo-tolerance and in parallel drug-resistance and radio-tolerance. Heat treatment can also lead to a multi-drug resistance.

Non-temperature dependent effects (mainly electromagnetic field stresses) could also produce chaperone-synthesis. The HSP manifestation in the biopsies could give a good clinical indication for the treatment response.

On the other hand, the chaperone HSP70 assists to freeze the actual dynamic equilibrium (the "status-quo") and so try to re-establish the cellular communication in the extra-cellular electrolyte. It is shown that their expression on the cell-membrane gains the apoptotic signals and enhances the immune reactions. HSP participates in the activation of the p53 tumor-suppressor and has been associated with the tumor-suppressor retinoblastoma protein.

The HSP could penetrate through the membrane, and interfere with the immune-system in the extracellular electrolyte. The membrane relocalization of HSP70 promotes the apoptosis, and has very important role (more than chaperone) in the membrane "fluid" to keep it functional. Tumor-specific membrane localization of HSP70 is mainly localized in the cholesterol-rich micro-domains of the membrane and well activates the NK-cells in immune response. Over-expression of HSP70 reduces the dysfunction caused by ischemia-reperfusion in myocardial ischemia and could play an important role in ischemia reperfusion. A broad band (0.2-20 MHz) electromagnetic field increased the HSP70 expression and so made some degree of ischemic protection. Produce the same increase of HSP70 expression by temperature, the perturbation should have been 14 orders of magnitude greater ! This is the great advantage of the non-temperature dependent effect of electric fields. The role of extracellular HSP is the topic of increasing interest of the overall immune reactions of the bio-systems.

The cellular lyses and the liberation of toxins of course could cause limits of the distortion process. However, the apoptotic cell-death or any systemic immune-action would be more natural and free from toxic complications. The thermally induced apoptosis and the activation of natural killer cells are both possible to solve this task.

Thus it would be highly useful in cancer treatment to provide a possibility to support the immune system to easily recognize the tumor cells and especially such tumor cells which express a reduced number of recognizable structures or which even hide their recognizable structures which could be recognized by APCs. Such a possibility would also allow to successfully treat metastases and patients which developed metastases and which are normally incurable.

Objective of the present invention is to provide the afore-mentioned possibility.

This objective is solved by the teaching of the independent claims. Further advantageous features and embodiments are evident from the description, the examples and the dependent claims.

### Description of the invention

Surprisingly it was found that common immune stimulants are highly useful for treatment of primary cancer and its metastases or for prevention of relapse of the cancer disease when used in association with radiofrequency waves which are also called radiofrequency signals or abbreviated with just radiofrequencies.

Thus, the present invention relates to an immune stimulant for treatment of primary cancer and its metastases or for prevention of relapse of the cancer disease when used in association with radiofrequency waves using capacitive coupling.

The present invention provides the possibility to actually prevent the development of cancer also in patients whose TSA was recognized by APCs but the cancer can not be indicated by any presently available imaging methods and also it provides a prevention of relapse of a malignant disease after a successful cancer treatment which common methods such as chemotherapy or radiotherapy and in addition provides a possibility to treat metastases and especially such metastases which are not recognized by the immune system and which unpreventable lead to death. Especially these cancer patients which had developed incurable metastases can now be cured.

Now state of the art drug or device or method is able to achieve this goal treatment of incurable cancers and also those which had developed metastases and also prevention of relapse of a previous and successful treated cancer disease.

Thus the present invention is a revolution in cancer treatment especially of incurable kinds of cancer.

The use of hyperthermia in cancer treatment is well known in the state of the art. However the activation of the adaptive immune system of the patient in a way that a highly personalized, in situ vaccination occurs was never reported in the state of the art literature.

Therefore the present invention also relates to a vaccine composed of at least one immune stimulant and radiofrequency waves in capacitive coupling for treatment of primary cancer and its metastases or for prevention of relapse of the cancer disease. The combination of at least one immune stimulant and radiofrequency waves using capacitive coupling is useful for treatment of primary cancer and its metastases or for prevention of relapse of the cancer disease.

This multidisciplinary method is able to cause an in-situ, in vivo, personalized tumor vaccination and to enable the immune system and especially the adaptive immune system to recognize and eliminate cancer cells and especially single cancer cells before a cancer disease occurs and to eradicate metastases and to recognize and eliminate new cancer cells after successful cancer treatment when the cancer relapses. This possibility to recognize cancer cells and cancer metastases of the immune system which is achieved by the present invention enables the immune system to kill the cancer cells and thus finally leads to successful treatment of primary cancer and its metastases and prevention of relapse of a malignant metastatic disease

In general vaccinations work using the natural immune system, "educated" and "trained" by outside immune stimulants. The "vaccination" of the present invention is the applied RF field. It is physical and not chemical stimulant of the immune system, but the philosophy is the same as the very first vaccination ever, done by Edward Jenner with cowpox against smallpox in humans.

The mode of action of the vaccine of the present invention could probably be explained as outlined in the following. The vaccine of the present invention stimulates the immune system in a more specific manner in comparison to immune stimulant alone. The malignant lesion is not attacked by the innate immune system, and the adapted one is not able to recognize the tumor, so also remain inactive. This is the reason, why simple immune stimulation can not be effective to destroy the developed malignancy. Hyperthermia alone (like monotherapy) is an immune stimulator when applied systemically, so faces the same complications as the other immune stimulants. The local hyperthermia makes necrosis or local effects, but the mean malignant force the dissemination of the cells and the metastatic formation is out of the scope of this treatment. The vaccine of the present invention is gaining the abscopal (or bystander) effect and attacks the malignancy in the system. In addition the elevated temperature distributes tumor-specific antigens on the surface of various tumor cells and assists in their secretion into the extracellular fluid, thereby triggering the immune reactions against the malignant cell (see Fig. 1).

Thus the vaccine of the present invention is also highly useful for treatment of primary cancer and its metastases and for prevention of relapse of the cancer disease in patients with weak immune system or with suppressed immune system due to the temperature increase in the body and preferably the whole body which as part of the invention triggers the immune response and activates the immune system.

Other tumor vaccines are not able to achieve such a result. In the following a summary of the status of current tumor immunotherapy possibilities, especially antitumor vaccination methods is given. The definite categorization of immunotherapy was established in the last decade, when the autologous immune response to target tumors came up.

A review on active immunotherapies (the passive one is the treatment with monoclonal antibodies) was named a cancer vaccination in 2007 (Vaccine was named due to the consortium leaded by Sabine Vaccine Institute). Below nine, anyway failed clinical case studies were presented. The field of vaccine immunotherapy shows numerous failures and so far relatively minimal benefits. Below are collected some clinical trials (Table 1: Matteo Vergati, Chiara Intrivici, Ngar-Yee Huen, Jeffrey Schlom, and Kwong Y. Tsang; (2010) Strategies for Cancer Vaccine Development; Journal of Biomedicine and Biotechnology, Volume 2010, Article ID 596432, 13 pages; doi:10.1155/2010/596432) showing the present possibilities.

**Table 1: Clinical studies with tumor-vaccination**

| VACCINE | PHASE | TUMOR | PATS. No. | NOTE |
|---|---|---|---|---|
| **Vaccines with viral vectors** | | | | |
| PSA-TRICOM | II | Prostate | 122 | 8.5 m OS, improvement vs. placebo |
| | II | Prostate | 32 | >16.4 m OS, improvement in HPS>18 m group |
| PANVAC-VF | III | Pancreas | 255 | Failed >OS Pts.with life expectancy <3m |
| **Vaccines with peptides** | | | | |
| Provenge | III | Prostate | 512 | 4.1 m OS improvement vs. placebo |
| Oncophage | III | Melanoma | 322 | Prolonged OS in M1a and M1b subpopulation |
| | III | Renal | 818 | No differences in DFS and OS |
| gp100:209-217(210M) | III | Melanoma | 185 | Significant improvement in RR and PES |
| Stimuvax | IIB | Lung | 171 | 17.3 m OS, improvement vs. BSC in locoregional stage IIB |
| **Vaccines with tumor cell or tumor-cell lysate** | | | | |
| OncoVAX | III | Colon | 254 | Significant improvement in DFS and OS in stage II |
| Reniale | III | Renal | 558 | Significant improvement in DFS and OS |
| GVAX | III | Prostate | 626 | Failed to improve OS vs. docetaxel |
| | III | Prostate | 408 | Failed. Higher death-rate in combination arm (vaccine + docetaxel) vs. docetaxel alone |
| **Vaccines with RNA** | | | | |
| mRNA from Pca cell-lines | I/II | Prostate | 19 | Immunological responses |

Multiple immuno vaccinations have been developed (like these listed in Table 1), or the very complicated off-situ method, as well as the robotic DC-culturing.

The vaccination of the present invention is superior over the known state of the art vaccinations. The present invention relates to an in-situ vaccination and makes advantage of the following effects:
1. The amplitude modulated (AM) RF electric field induces immunogenic cell death
   Proofs:
   1.1. Apoptotic cell death with apoptotic body formation
   1.2. Immunogenic DAMP/SAMP formation:
      1.2.1. HSPs overexpression and externalization
      1.2.2. DR5 (TRAIL) overexpression
2. Immunogenic cell death induced by RF waves creates strong local immune reaction against the tumor. The unhidden tumor antigens became recognizable by APCs. RF waves induced immunogenic cell death loads the patient unique TSA into the DC by phagocytosis and the apoptotic bodies create specific immune reactions against the patient's malignancy.
   Proofs:
   2.1. Leukocyte invasion ring around the destroyed tumor tissue
   2.2. Presence of the T cells in this ring
3. The immune stimulants such as bacteria derived immune stimulants (like for example Picibanil, Ancer, C.parvum, Coley-extract, other LPS-extracts, etc.) and the RF wave induced local effect is systemic, thereby destroying the far metastases by TSA loaded DC activated CTLs.
   Proofs:
   3.1. LPS study, human abscopal effects, experimental abscopal (bystander) actions

The vaccine of the present invention causes a systemic effect (the adaptive immune system acts in the whole body) and this is one of the most important advantages of the present vaccine, namely to make a local treatment systemic. The systemically acting vaccine of the present invention discovers the hidden antigens and treats cancer without causing extreme artificial fever. The inventive vaccine makes this immune support without inducing artificial fever and without any extra load on a weak patient.

Thus the present invention relates to an immune stimulant useful for the treatment of primary cancer and its metastases even in disseminated cell states, which can not be detected by state of the art imaging methods and useful for prevention of relapse of the cancer disease when or under the condition that radiofrequency waves are present and that capacitive coupling in a condenser arrangement are used. The immune stimulant under this condition causes a systemic vaccination activating the immune system and especially the adaptive immune system without causing artificial fever which is especially useful for the treatment of metastases and primary cancer which cannot be detected by state of the art methods as well as especially useful for the prevention of relapse of a successfully treated cancer.

Such an effect cannot be achieved only by methods for immune stimulation. In accordance with the present invention the following immune stimulations can be used:
4.1. Systemic (sc. im.) administration of immuno-stimulant (secondary generated cytokines will activate the immune system)
4.2. Local (intratumoral) administration of immuno-stimulant (direct local immuno-stimulation via TLR pathway, cross-presentation, and secondary generated cytokines stimulation)
   4.2.1. Intratumoral injection
   4.2.2. Targeted compound delivery to tumor by liposomes (electro-sensitive liposomes)

The systemic effect generates strong immune memory against the tumor, preventing the relapse of the cancer disease. The prophylaxis in its classical meaning is not true, without presence of malignant cells which are providing the possibility to unhide their TSA, the method does not work. However it has a prophylaxis like effect: most of the tumors have definite area creating metastases. For example colorectal cancer or pancreatic cancer creates metastases in liver in >90%. In these cases the place of likely-to-be-metastatic could be treated locally, even when the primary tumor is operated out. The inventive vaccine could be applied when no imaging proof of the metastasis exist, but forming of metastasis or relapse is likely. This is important is most of the cases of the primary tumor treatment, even after complete remission. The complete remission as clinical response does not mean complete cure, only means that the present imaging systems can not see the malignancy at that particular place and particular time.

In theoretical point of view the full process the inventive vaccine acts is simple. We have to look at the process not from the treatment side, but form the molecular side of the process emphasizing the HSP, DR5, apoptotic body, TSA, CTL, NK, etc. cellular "reagents" as the basis of the invention.

In comparison to the known RFA method the most important differences are summarized in the following:
RFA method:
   - uses short wavelength and high frequency alternating current [130 MHz-2400 MHz]
   - is an invasive method (intratumoral insertion of needles)
   - only local treatment is possible
   - the lesion is burned out / the tumor is burned
   - only heat causes the therapeutic effect
   - causes vehement necrosis
   - uses "antennas" in form of needles
   - uses radiative coupling
   - the needles are guided by ultrasound or imaging methods such as X-ray screening, CT scan within the patient's body
   - is only useful for solid tumors

In contrast the vaccination method of the present invention:
- uses RF waves of preferably 13.56 MHz, but not more than 50 MHz
- is not invasive
- makes systemic treatment possible
- uses heat, immune stimulants and the activated patient's own immune system to fight against the cancer cells and thus makes use of an synergistic effect of the heat with the patient's immune system
- causes apoptosis
- does not cause necrosis
- uses a condenser arrangement
- uses capacitive coupling
- does not require support by ultrasound or any imaging method
- is especially useful to destroy single cancer cells and thus to treat cancer at the very beginning in its very initial state and also to treat the relapse of a cancer disease at the very beginning in the very initial state
- induces the ability in the immune system and triggers the adaptive immune system to recognize the cancer cells thus establishing a long-term memory in the adaptive immune system to recognize the cancer cells, thus providing a vaccination against the cancer cells. This might be the most important difference to all known cancer treatment methods.

It has to be understood that the head generated by the RFA method is different from the heat generated by the inventive vaccination. Temperature and heat are different quantities. Temperature is not a quantity, not proportional to mass or volume. Temperature characterizes the equilibrium only. Heat is active energy, which partly increases the temperature, partly modifies chemical bonds and molecular structures, which is the aim of the present inventive vaccine. For example, we are eating (absorbing energy from the food measured in kj) not for increasing our body temperature. The fact that heat is also measured in kj does not indicate identical physical parameters.

Furthermore the present invention is directed to immune stimulants for treatment of metastases even in the cases when the present imaging methods are not able to detect these; or for prevention of relapse of the cancer disease, wherein the immune stimulant is applied in association with radiofrequency waves using capacitive coupling. A further aspect of the invention is related to an immune stimulant for treatment of metastases or for prevention of relapse of the cancer disease, wherein the immune stimulant has strong synergy with the radiofrequency waves.

The radiofrequency waves can be generated by any conventional RF hyperthermia device using radiofrequency (RF) waves in a condenser arrangement of at least two electrodes which are preferably equipotential over their total surface, using RF-current of preferably 13.56 MHz. The at least one RF-electrode and the at least one counter electrode are the electromagnetic energy transfer means which are part of a condenser for directing energy to a target.

The RF hyperthermia device applied in the present invention uses capacitive coupling, inductive coupling or radiative coupling and preferably capacitive coupling and alternating current (AC) and radio frequency (RF) waves.

The radiofrequency (RF) used by the RF hyperthermia device is low and does not exceed 50 MHz. In contrast, the radiation hyperthermia devices have to use a high frequency of at least 100 MHz, otherwise accurate focusing is impossible. Generally, the antenna (radiative) has to be optimized to 50 Ohm (this is the accepted standard). This function is made by the tuner. In the present invention the low frequency of preferably 13.56 MHz or 6.78 MHz or 27.12 MHz or 40.68 MHz or any value in between is preferred. In contrast common radiative hyperthermia uses short wavelength and high frequency in the range of 70 MHz - 2400 MHz.

The radiofrequency (RF) hyperthermia device itself comprises at least a radiofrequency source, an amplifier, a sensor, optional a feedback amplifier and optional a modulation signal generator. Suitable RF hyperthermia device are for example disclosed in the US application 13/123,838 or the US application 12/863,418. The RF hyperthermia device used within the present invention is quite different from the hyperthermia devices of the state of the art as outlined in the following.

A state-of-the-art hyperthermia device is described in US 2004/0230263 A1. It differs from the RF hyperthermia device applied in the present invention in the following features: In the device of US 2004/0230263 A1 dipole antennas (radiative coupling) are used. Radiative RF is applied through the patient or more precisely through the target tissue by using absorbed RF radiation. In the radiative solution the target is independent from the circuit, the feedback is made by the standing-wave-ratio (SWR) only, which measures the reflected power in comparison to the forwarded. The RF hyperthermia device applied in the present invention does not use dipole antennas, a condenser arrangement is used, wherein the patient's body between the electrodes is the dielectric material which is part of the conductive circuit. This enables a direct control of the target as a part of the circuit, and generates a more precise and accurate feedback for controlling the process. The RF hyperthermia device applied in the present invention uses condenser electrodes (capacitive coupling) for the application of RF waves through the respective body cross section so that a systemic treatment is obtained. This conventional hyperthermia device induces phase-shifted interference between the antennas and interference of their standing wave radiation in order to tune the focus on the desired area. The present invention uses conductivity differences of the respective tissues (e.g. malignant tumor tissue has a higher conductivity than healthy tissue), thus leading to an automatic selection of the focus to the malignant tumor tissue. This has immediate consequences on expansible organs like the lung or the heart, or if the patient moves during a treatment session which may exceed one hour. While the focus in the conventional device remains at the spot on which it was focused before, independent from the actual position of the tumor, the present invention follows any movement of the target because the RF current automatically flows in the correct direction. In this conventional hyperthermia device the target is treated like an electrically independent object absorbing the radiated energy thereby causing burns and vehement necrosis. The present invention uses the target as a part of the electric circuit, as a dielectric material of a condenser in a resonant circuit. Consequently, the heating process is carried out and controlled in a different fashion. This conventional hyperthermia device uses SAR (specific absorption rate) absorbed energy as the only heating mechanism for achieving a beneficial effect, thus by heating up the tumor, the tumor cells are burned. The present invention uses Joule heat (Q=I²R) by converting the current flow into heat as well as the potential difference for an electric field effect, thereby causing apoptosis in the cancer cells and thus triggering the immune system to an immune response. Thus the patients own immune systems starts fighting against the recognized single cancer cells, wherein the fight is supported by the administered immune stimulants thus becoming an effective cancer therapy especially for the very first initial stadium of cancer development which is undetectable by any known diagnostic method and for the treatment of metastases due to the fact that the treatment is systemic and not local as in the state of the art. The conventional hyperthermia device controls temperature only as a tool for reproducing and standardising the therapy. In contrast, the present invention uses the absorbed energy (J/kg) and the conductivity of the patient (S=I/R) for strict control of the therapy conditions. This conventional hyperthermia device implicitly assumes that the success of the therapy depends only on the heat effect relative to the achieved temperature. By such a method mainly necrosis is caused in the target tissue. The present invention, however, does not require achieving such high temperatures at which necrosis occurs, because the field effect causes apoptosis at lower temperatures. Thus the RF hyperthermia device applied in the present invention treats tumorous or malignant tissue, cancer, tumours and especially metastases and single cancer cells by inducing and/or causing apoptosis and by enabling the immune system to recognize the cancer cells and to start fighting against them, while common devices using radiative coupling induce necrosis and are not even able to treat metastases and single cancer cells in the initial state of cancer development or the initial state of cancer relapse. The RF hyperthermia device applied in the present invention does not use radiative coupling and uses capacitive coupling, wherein the patient is the dielectric material or dielectricum as part of the electric circuit.

The differences can be summarized as follows:
1. RFA devices use RF-radiation/absorption
2. The inventive vaccination uses RF current conduction
3. RFA devices use a needle as antenna for radiative coupling
4. The inventive vaccination uses electrodes for capacitive coupling
5. RFA devices requires high frequencies (above 130 MHz)
6. The inventive vaccination requires frequencies below 50MHz and preferably 13.56 MHz or 6.78 MHz or 27.12 MHz or 40.68 MHz 13.56 MHz and most preferably 13.56 MHz
7. RFA devices cause necrosis (no vaccination possible)
8. The inventive vaccination causes apoptosis and recognition of cancer cells and thus causes a vaccination
9. RFA devices are use invasively and locally
10. The inventive vaccination is used not invasively and systemically
11. RFA devices are only useful to treat solid tumors
12. The inventive vaccination is especially useful to treat isolated and widespread cancer cells such as metastases and the initial stadium of cancer development

All common immune stimulants can be used in the present invention. Preferred examples are selected from the group comprising or consisting of bacterial preparations like lipopolysaccharides, Picibanil (OK-432), Ancer (Maruyama vaccine) and killed *Corynebacterium parvum* bacteria and its extract, cytokines such as IL and GM-CSF, or any TLR receptor agonist agents (bacterial or herbal or synthetic) and any natural or synthetic agent acting the TLR pathway.

These immune stimulants could be directly injected into the tumor lesion, or directed by special tumor targeting methods (such as liposome carrier, magnetic targeting, nanoparticle carriers, etc.). This directed targeting strongly intensifies the cross-priming, when the effectors (e.g. the bacterial endotoxins, like LPSs or LPS-like materials acting through the TLR receptor pathways). These are bonded to the APC cells (e.g. DC) together with the TSAs inducing stronger and longer effective immune reactions. In case of liposome delivery some vaccine adjuvants (like aluminium oxide (alumina), zeolite or some traditional oriental medicine herbal effectors) could be added.

The common immuno-therapeutic vaccination methods are applicable only in very limited conditions by the state of art. The modality faces with various requests of the modern era. It has to be:
- Effective
- Personalized (specific)
- Applicable for all the tumor-lesions
- Blocks the relapse and metastases
- Simple applicable
- Not too expensive

These criteria are not fulfilled by the state of the art tumor vaccination methods. The applied therapies were not effective in general, only in some special fields. The main reason of the failures and low efficacy is the low immunogenic activity of the tumor cells in general cases. The immune system did not recognize the tumor cells, and thus was not active to eliminate them. The immune stimulation without specific immune identification processes (e.g. cytokine therapies with IL-2, IL-8, TNF, etc.) could far not reach the desired efficacy. Most of the therapies are working off-situ, taking the immuno-potential form the patients or other healthy subjects and giving the off-situ manipulated, labor-made vaccine back to the patient.

The vaccine, the immune stimulant as well as the vaccination in accordance with the present invention solve the above-mentioned problems. The present invention provides immune stimulants, vaccines and vaccination methods for the primary cancer as well as for the treatment of metastases and also for the prevention of relapse of the cancer disease especially after a successfully treated cancer disease.

The goals of the present invention are outlined in the following after the explanation of the nomenclature.

The term "**In vivo**": Antigens for immunization is generated in vivo in the patient's body. Most of the tumor-vaccination manipulates with artificially produced antigens or using the patient's own antigens which is prepared in off-situ, in-vitro laboratory conditions and reinjected to the patient later. In-vio means in our nomenclature, that the appropriate antigen forming is made inside of the patient's body, so that not any invasive process is involved.

The term "**In-situ**": According to the invention, the immunization process is carried out in the site of the tumor. In most of the "classical" tumor-vaccination the antigens are placed in the body far away from the place of the expected active molecular actions. The in-situ nomenclature means that the immunization, i.e. the recognition of the specific TSA is processed actually in the tumor.

The term "**Personalized**": Immunization with the patient's own TSA (tumor specific antigen). As we mentioned above, most of the tumor vaccination uses artificial antigens, in many cases far away from the patient's own tumor specific (TSA) or tumor associated (TAA) antigens. In most of the cases the major challenge is using general (non-personalized) proteins or protein-cocktails for immune effects, which could be effective for some patients, but far not for all patients. These conditions make the treatment uncontrollable and unpredictable. In accordance to the invention, the vaccination uses the own unique protein molecule pattern of the patient and not artificial immune agents. The inventive vaccination creates the patient's own, very individual and incomparable TSA "cocktail" for actual and precise immune identification. In the state of the art such a personalized, simple and cheap tumor vaccination does not exist. The existing processes are too sophisticated, highly complicated, very expensive and rarely effective. For example the TSA could be obtained from the out-operated specimen. After its in-vitro recognition process with dendritic cells (DC) and after special immune activation the "vaccine" is injected directly into the tumor or systemically administered to the patient. The inventive vaccination induces specific characteristic highly immunogenic cell death. This process has various sub-processes.

The term "**Apoptosis induction**": The inventive vaccine can induce special *immunogenic apoptosis* with apoptotic body formation. Apoptotic bodies (loaded with tumor cell components, including the patient's very own TSAs) can be phagocytized easily by the appropriate APC (antigen presenting cells, DCs). Research results in the past show a major immuno-stimulative effect of the stress-induced apoptosis. The apoptotic bodies produced in this process contain a large amount of HSPs together with the specific TSA. Their phagocytosis by APC induces strong and specific immune reactions.

The term "**DAMP** (SAMP)": The inventive vaccine can induce unique molecular changes (DAMP/SAMP) in the tumor cells what can be immune stimulative and on the other hand can promote the immune recognition of the tumor cells.

The term "**HSP overexpression**": HSP overexpression and externalization refers to the following. HSP chaperone proteins have important and primary role in tumor immunology processes. The HSPs are able to directly connect to the membrane receptors of immune cells (DCs, macrophages), thereby activating them. On the other hand the HSPs help recognizing the TSAs by cross-priming processes, where the HSPs bonding to APCs together with TSAs induce strong specific immune reactions. HSPs are able transporting TSAs from the cell and promote recognizing it by DC cells.

The term "**TRAIL (DR5) overexpression**": According to many publications this molecule has key importance in the antitumor immune reactions. Tumor distortion generated by the inventive vaccine makes such damage associated molecular pattern (DAMP) and stress associated molecular pattern (SAMP), which result in major tumor specific immune reactions by the liberation of the patient's own tumor specific antigens (TSA). In this way the vaccination is in-situ, no labor manipulation is necessary for its success. The inventive vaccination fulfills all the above listed requirements of vaccinations, especially when applied together with other immune stimulative processes. The inventive vaccination affects systemically (abscopal or bystander effect) and the treatment is active on the disseminated malignant cells and on the far distance formed metastases too, especially when applied together with other immune stimulative processes. The induced immune reaction also forms proper immune memory, which blocks the relapses of the disease, i.e. the cancer disease.

Advantages of the inventive vaccine are summarized as follows:
- Immunogenic apoptotic body formation
- TSA-HSP cross priming
- Effective antigen recognition by APCs
- APC cell activation and maturation
- Specific cytotoxic T cell activation by APCs
- Destruction of distant metastases far away from the treated tumor
- Development of the specific immune memory preventing the tumor recurrence and the cancer relapse.

The inventive vaccination achieves effects which could not be reached by common cancer treatment strategies. The mode of action of the inventive vaccine could probably be explained as follows. The TSAs in apoptotic bodies produced by the inventive vaccination became reachable by APCs. Due to the identification of the TSA and the adjuvant HSP enrichment by the strong stress of the treatment with the inventive vaccine the APC induces remarkable immune reactions. These effects
1. Activate the CTL system obtaining the patient's specific TSA information. This allows to find and destroy the malignant cells in far distant metastases or in disseminated form.
2. Creates an effective immune memory blocking the later relapse of the malignancy.

The complete process is be well promoted by the immune stimulants, which form the local effect systemic. The abscopal (bystander) effect could be oriented and controlled. The immune stimulant activates numerous non-specific immune reactions as well (e.g. activity of NK-cells, activity of the phagocytosis, boosting some humoral immune-processes, etc.,), completing the tumor eradication in the system.

Treatment of the following primary cancer types can be achieved and metastases of the following cancer types can be treated and also relapse of the following cancer types can be prevented by the present invention: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer.

Moreover the present vaccine does not have and does not cause any serious side effects so that the inventive vaccination is very suitable to be combined with common cancer therapies such as chemotherapies with one or more of the following chemotherapeutic agents: actinomycin D, aminoglutethimide, amsacrin, anastrozol, antagonists of purine and pyrimidine bases, anthracycline, aromatase inhibitors, asparaginase, antiestrogenes, bexaroten, bleomycin, buselerin, busulfan, camptothecin derivates, capecitabin, carboplatin, carmustine, chlorambucil, cisplatin, cladribin, cyclophosphamide, cytarabin, cytosinarabinoside, alkylating cytostatics, dacarbacin, dactinomycin, daunorubicin, docetaxel, doxorubicin (adriamycin), doxorubicin lipo, epirubicin, estramustine, etoposid, exemestan, fludarabin, fluorouracil, folic acid antagonists, formestan, gemcitabin, glucocorticoides, goselerin, hormones and hormone antagonists, hycamtin, hydroxy urea, idarubicin, ifosfamid, imatinib, irinotecan, letrozol, leuprorelin, lomustin, melphalan, mercaptopurine, methotrexate, miltefosin, mitomycine, mitosis inhibitors, mitoxantron, nimustine, oxaliplatin, paclitaxel, pentostatin, procarbacin, tamoxifen, temozolomid, teniposid, testolacton, thiotepa, thioguanine, topoisomerase inhibitors, topotecan, treosulfan, tretinoin, triptorelin, trofosfamide, vinblastine, vincristine, vindesine, vinorelbine, antibiotics with cytotoxic activities.

### Abbreviations:

- APC:: antigen presenting cell
- CTL:: cytotoxic T lymphocyte
- DAMP:: Damage Associated Molecular Pattern
- DC:: dendritic cell
- DR5 (TRAIL):: death receptor (TNF-related apoptosis-inducing ligand)
- GM-CSF:: granulocyte-macrophage colony stimulating factor
- HMGB1:: high mobility group box
- HSP:: heat shock protein
- IL:: interleukin
- LPS:: lipopolysaccharide
- NK:: natural killer cell
- OTM:: oncothermia method
- SAMP:: Stress Associated Molecular Pattern
- TAA:: tumor-associated antigen
- TLR:: toll like receptor
- TNF:: tumor necrosis factor
- TSA:: tumor specific antigen

### Description of the figures

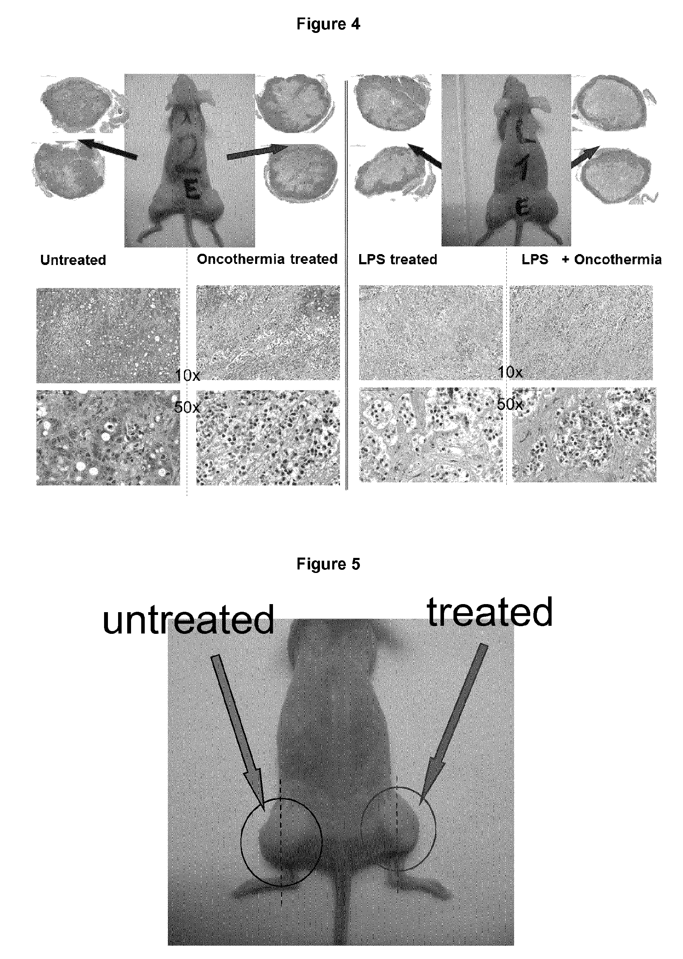

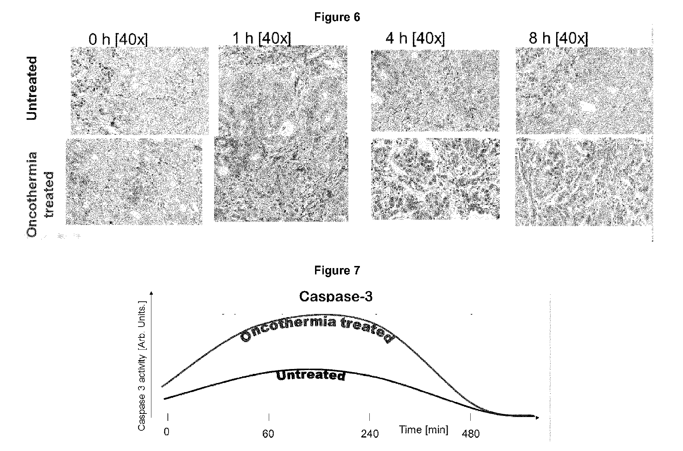

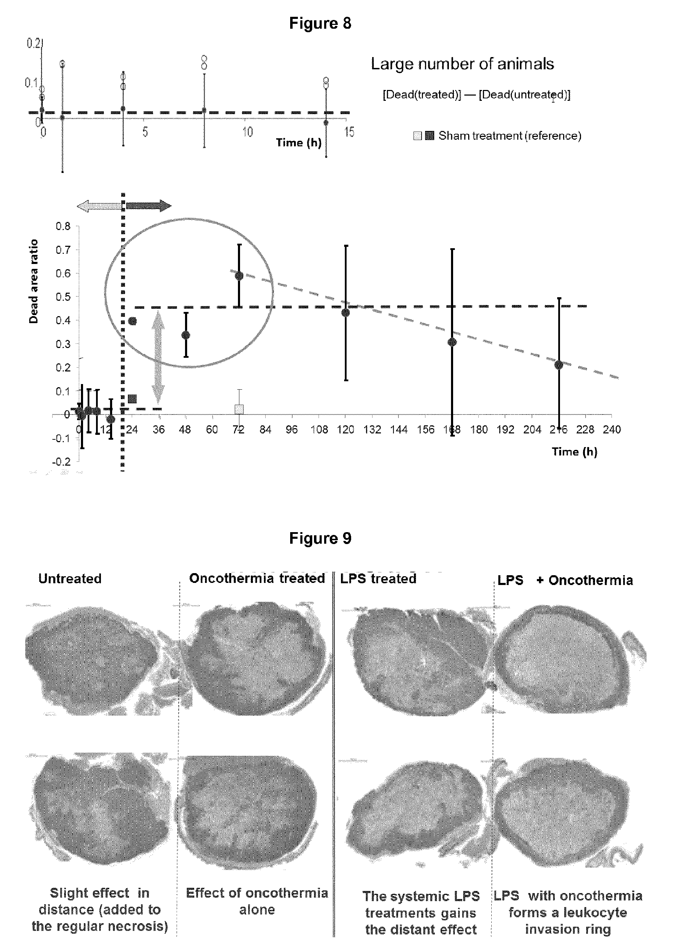

**FIGURE 1**
   Show how the RFA method works. An antenna needle is inserted into the solid tumor and heat is produced by the applied local RF current which burns the tumor and causes vehement necrosis. RFA is an invasive method, using RF current to produce heat and thus causing ablation by an antenna arrangement using radiative coupling.
**FIGURE 2**
   Shows that a clear invasion ring appears (arrows) around the tumor. Thus a strong immune reaction after treatment in accordance with the present invention occurs.
**FIGURE 3**
   HSP (heat shock proteins) productions measured on mRNS level (B is the treated tumour [black], A is the untreated one [gray], CTR is the untreated control. The gray-line is the unchanged level.
**FIGURE 4**
   Gives the proof that the local effect is also systemic. By LPS (lipopolysaccharide from E.coli, [100 µg LPS in 100 µl Salsol solution was used.) addition the effect of cell-killing is massive on the untreated far-distance tumor too. The LPS was systemically administered.
**FIGURE 5**
   The results were made on nude-mice with two, far-distance tumors. Only their right side was treated. By LPS addition the effect of cell-killing is massive on the untreated far-distance tumor too. The LPS was systemically administered.
**FIGURE 6**
   The inventive effect is shown in human-treatment, when the primary tumor is treated only, and the far-distance metastases disappear (HT29 human colorectal xenograft model, single shoot, 30 min, 42 C).
**FIGURE 7**
   The caspase-3 activity (important mark of the apoptosis signal-pathway) grows both in treated and untreated (far-away) tumors in nude mice.
**FIGURE 8**
   Time-series of nude mice xenograft (HT29 human colorectal), single shoot, 30 min, 42 C. Every point represents 3 double tumor-bearing animals. The sacrifice of the animals is made at the time after treatment (indicated on X-axis). The observation, that the apoptosis starts after 24 hours (the insert shows no effect in the first 8 hours) and the difference between the treated and far-away not treated tumor-death lowers, the action started far away from the local treatment.
**FIGURE 9**
   LPS assisted treatment, when the LPS is applied is enhances very much the effect on the far-away tumor (It is the enlargement of the results of Fig.5). The state of the art literature shows only off-situ immune support, when the laboratory assistance was necessary for far-distant (immuno-assisted) effects. In the treatment in accordance with the present invention the main point is the in-situ application.

## Claims

1. Immune stimulant for treatment of primary cancer and its metastases or for prevention of relapse of the cancer disease when used in association with radiofrequency waves using capacitive coupling.

2. Immune stimulant according to claim 1, wherein the immune stimulant is selected from the group comprising bacterial preparations, lipopolysaccharides, Picibanil, Ancer, killed *Corynebacterium parvum* bacteria and its extract, cytokines, TLR receptor agonist agents and any natural or synthetic agent acting the TLR pathway.

3. Immune stimulant according to claim 1 or 2 for vaccination of people with increased risk to develop cancer.

4. Immune stimulant according to claim 1, 2 or 3, wherein the metastases or the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer.

5. Vaccine composed of at least one immune stimulant and radiofrequency waves using capacitive coupling for treatment of primary cancer and its metastases or for prevention of relapse of the cancer disease.

6. Vaccine according to claim 5, wherein the immune stimulant is selected from the group comprising comprising bacterial preparations, lipopolysaccharides, Picibanil, Ancer, killed *Corynebacterium parvum* bacteria and its extract, cytokines, TLR receptor agonist agents and any natural or synthetic agent acting the TLR pathway.

7. Vaccine according to claim 5 or 6, wherein the vaccine is used for in-situ vaccination.

8. Vaccine according to claim 5, 6 or 7, wherein the metastases or the cancer is selected from the group consisting of: adenocarcinoma, choroidal melanoma, acute leukemia, acoustic neurinoma, ampullary carcinoma, anal carcinoma, astrocytoma, basal cell carcinoma, pancreatic cancer, desmoid tumour, bladder cancer, bronchial carcinoma, non-small cell lung cancer (NSCLC), breast cancer, Burkitt's lymphoma, corpus cancer, CUP-syndrome (carcinoma of unknown primary), colorectal cancer, small intestine cancer, small intestinal tumours, ovarian cancer, endometrial carcinoma, ependymoma, epithelial cancer types, Ewing's tumours, gastrointestinal tumours, gastric cancer, gallbladder cancer, gall bladder carcinomas, uterine cancer, cervical cancer, cervix, glioblastomas, gynecologic tumours, ear, nose and throat tumours, hematologic neoplasias, hairy cell leukemia, urethral cancer, skin cancer, skin testis cancer, brain tumours (gliomas), brain metastases, testicle cancer, hypophysis tumour, carcinoids, Kaposi's sarcoma, laryngeal cancer, germ cell tumour, bone cancer, colorectal carcinoma, head and neck tumours (tumours of the ear, nose and throat area), colon carcinoma, craniopharyngiomas, oral cancer (cancer in the mouth area and on lips), cancer of the central nervous system, liver cancer, liver metastases, leukemia, eyelid tumor, lung cancer, lymph node cancer (Hodgkin's/Non-Hodgkin's), lymphomas, stomach cancer, malignant melanoma, malignant neoplasia, malignant tumours gastrointestinal tract, breast carcinoma, rectal cancer, medulloblastomas, melanoma, meningiomas, Hodgkin's disease, mycosis fungoides, nasal cancer, neurinoma, neuroblastoma, kidney cancer, renal cell carcinomas, non-Hodgkin's lymphomas, oligodendroglioma, esophageal carcinoma, osteolytic carcinomas and osteoplastic carcinomas, osteosarcomas, ovarial carcinoma, pancreatic carcinoma, penile cancer, plasmocytoma, squamous cell carcinoma of the head and neck (SCCHN), prostate cancer, pharyngeal cancer, rectal carcinoma, retinoblastoma, vaginal cancer, thyroid carcinoma, Schneeberger disease, esophageal cancer, spinalioms, T-cell lymphoma (mycosis fungoides), thymoma, tube carcinoma, eye tumours, urethral cancer, urologic tumours, urothelial carcinoma, vulva cancer, wart appearance, soft tissue tumours, soft tissue sarcoma, Wilm's tumour, cervical carcinoma and tongue cancer. Particularly suitable for treatment are, for example, astrocytomas, glioblastomas, pancreatic cancer, bronchial cancer, breast cancer, colorectal cancer, ovarian cancer, gastric cancer, laryngeal cancer, malignant melanoma, oesophageal cancer, cervical cancer, liver cancer, bladder cancer, and renal cell cancer.

9. Combination of at least one immune stimulant and radiofrequency waves in capacitive coupling for treatment of primary cancer and its metastases or for prevention of relapse of the cancer disease.

10. Method for treatment of primary cancer and its metastases and for prevention of relapse of the cancer disease comprising administering to a patient a combination of at least one immune stimulant and radiofrequency waves.
